# EUROPÄISCHE PATENTSCHRIFT

(11) **EP 3 064 234 B1**
(45) Veröffentlichungstag und Bekanntmachung des Hinweises auf die Patenterteilung: **03.01.2018**
(21) Anmeldenummer: 16160496.2
(22) Anmeldetag: 29.05.2008
(51) Int. Cl.: A61M 1/36

(54) **HERZ-LUNGEN-MASCHINE**
HEART-LUNG MACHINE
MACHINE COEUR-POUMON

(43) Veröffentlichungstag der Anmeldung: 07.09.2016
(62) Teilanmeldung aus: 08760191.0
(73) Patentinhaber: Sorin Group Deutschland GmbH, 80939 München (DE)
(72) Erfinder: HAHN, Andreas, 82335 Berg (DE); KNOTT, Erwin, 85586 Poing (DE)
(74) Vertreter: Hoffmann Eitle

(56) Entgegenhaltungen:
- EP-A- 1 767 232
- WO-A-96/25972
- US-A- 5 429 058
- US-A- 6 071 258
- US-H- H1 324

## Beschreibung

### Technisches Gebiet

Die Erfindung betrifft eine Herz-Lungen-Maschine, umfassend eine Mehrzahl medizinischer Geräte, ein Bedienpaneel, zwei oder mehr sich im Wesentlichen vertikal erstreckende Seitenelemente und ein sich zwischen den Seitenelementen und bevorzugt im Wesentlichen horizontal erstreckendes Verbindungselement, wobei das Bedienpaneel separate Bedienelemente zum Bedienen der medizinischen Geräte aufweist, das Verbindungselement die Seitenelemente miteinander verbindet, wobei das Verbindungselement und die Seitenelemente einen Rahmen zum Tragen der medizinischen Geräte bilden, so dass die medizinischen Geräte zumindest teilweise zwischen den Seitenelementen aufnehmbar sind, und wobei das Bedienpaneel über eine Grundfläche der medizinischen Geräte auskragend ausgebildet ist.

### Stand der Technik

Im Gesundheitswesen werden Lebenserhaltungssysteme, wie insbesondere Herz-Lungen-Maschinen zur Unterstützung oder auch als Ersatz für körpereigene Vitalfunktionen, beispielsweise Kreislauf, eingesetzt. Die seit Jahrzehnten bekannten Lebenserhaltungssysteme wurden in der Vergangenheit ständig weiterentwickelt und verbessert. Dabei wurde beispielsweise die Herz-Lungen-Maschine auf verschiedenste Art und Weise gestaltet, indem die Komponenten, wie Blutpumpen, Oxygenator, Wärmetauscher, Bedienelemente, Stromversorgung, Befestigungselemente usw. hinzugefügt und unterschiedlich angeordnet wurden. Die verschiedenen Gestaltungen verfolgten das Ziel, die Komponenten effizient zu platzieren und gleichzeitig das Lebenserhaltungssystem einfach bedienbar zu machen. Im Zuge der universellen Einsetzbarkeit traten dabei solche Lebenserhaltungssysteme immer mehr in den Vordergrund, die eine Vielzahl medizinischer Geräte umfasst. Eine weitgehend intuitive und somit fehlerunanfällige Bedienbarkeit eines solchen Lebenserhaltungssystems gewinnt daher mit der zunehmenden Zahl von Geräten immer mehr an Bedeutung. Aufgrund der Vielzahl medizinischer Geräte und der damit verbundenen Bedienelemente sowie Peripheriegeräte wird die Bedienbarkeit bei gleichzeitiger voluminöser Einschränkung des Gesamtkomplexes zu einer wichtigen Voraussetzung bei der Konstruktion von
Lebenserhaltungssystemen.

Um diesem Erfordernis gerecht zu werden, wird in der DE 197 23 671 A1 eine Herz-Lungen-Maschine mit mehr als zwei Blutpumpen beschrieben, bei der eine spezielle Anordnung der Blutpumpen einer einfachen Bedienbarkeit der Herz-Lungen-Maschine dient. Hierin wird beispielsweise beschrieben, dass die Blutpumpen nicht in einer Reihe nebeneinander, sondern um den Oxygenator oder Wärmetauscher herum angeordnet werden. Dadurch wird eine möglichst kompakte Bauweise der Herz-Lungen-Maschine ermöglicht.

Nach wie vor besteht jedoch das Problem der Übersichtlichkeit bei einem Lebenserhaltungssystem mit einer Mehrzahl medizinischer Geräte, so dass eine Fehlbedienung, die im Einsatz des Lebenserhaltungssystems zu fatalen Folgen führen kann, hierdurch nicht ausgeschlossen werden kann.

Des Weiteren sollen bei der Konstruktion von professionell eingesetzten Geräten auch arbeitsmedizinische Gesichtspunkte stärker beachtet werden. Dies soll weitgehend verhindern, dass Haltungsschäden oder andere auf eine andauernde Benutzung eines professionell angewendeten Gerätes zurückzuführende gesundheitliche Probleme beim Bedienpersonal auftreten. Als Folge dieser Anforderung ist es bei einer Konstruktion eines Gerätes, das professionell eingesetzt werden soll, von steigender Bedeutung, dass die Bedienung des Gerätes nicht nur möglichst intuitiv, sondern auch bedienerfreundlich im Sinne einer ergonomischen Ausgestaltung konstruiert werden.

Dieser Aspekt ist bei den bisherigen Lebenserhaltungssystemen nahezu vollständig außer Acht gelassen worden, und stellt eine weitere Herausforderung an die Konstruktion eines Lebenserhaltungssystems dar.

Aus WO 96/25972 A1 ist eine Kardioplegievorrichtung bekannt, die einem Patienten ein variables Verhältnis von Blut und Kardioplegielösung zuführt. Sie umfasst zwei separate Pumpen.

### Darstellung der Erfindung

Der Erfindung liegt die Aufgabe zugrunde, einen dem technischen Gebiet zugehörigen Gerätekomplex zu konstruieren, der ohne erhöhten Platzbedarf die Bedienbarkeit bekannter Geräte verbessert.

Die Lösung der Aufgabe wird durch die Merkmale des Anspruchs 1 angegeben. Erfindungsgemäß ist das Lebenserhaltungssystem, d.h. die Herz-Lungen-Maschine, somit dadurch gekennzeichnet, dass eine ausgezeichnete Bedienposition dadurch ausgezeichnet ist, dass das Bedienpaneel derart ausgebildet ist, dass jedem Gerät ein mit nur diesem Gerät und mit der Bedienposition fluchtendes Bedienelement zugeordnet ist. Die Grundfläche der Mehrzahl medizinischer Geräte ist dabei ohne die Grundfläche des Bedienpaneels anzusehen, so dass das flächig ausgedehnte Bedienpaneel als über diese Grundfläche auskragend ausgebildet beschrieben werden kann. Jedes Gerät wird dabei durch ein Bedienelement bedient, das mit diesem Gerät und der Bedienposition fluchtend angeordnet ist. Das Bedienelement kann dabei auch ein Bedienmodul sein, das mehrere einzelne Bedienelemente umfasst, wobei das Bedienmodul hauptsächlich, insbesondere ausschließlich zur Bedienung des mit ihm und der Bedienposition fluchtend angeordneten Gerätes dient. Es können auch Bedienelemente zur Bedienung von mehr als einem Gerät in einem Bedienmodul enthalten sein. Beispiele für Bedienmodule und Bedienelemente sind Dreh- oder Schieberegler, Schalter, Taster oder Touchscreens. Die Fluchten der Mehrzahl medizinischer Geräte laufen somit im Wesentlichen in der Bedienposition zusammen.

Durch die erfindungsgemäße Ausgestaltung des Lebenserhaltungssystems ist durch die fluchtende Anordnung der Bedienelemente auf dem Bedienpaneel mit den medizinischen Geräten, ausgehend von der ausgezeichneten Bedienposition, eine intuitive Zuordnung von Bedienelement und bedientem Gerät gewährleistet. Zwar lässt sich das Lebenserhaltungssystem von verschiedenen Positionen aus bedienen, eine ausgezeichnete Bedienposition wird jedoch durch die erfindungsgemäße Konstruktion des Lebenserhaltungssystems, insbesondere durch einen Schnittbereich der Fluchten definiert.

Mit Vorteil sind die Bedienelemente nebeneinander auf dem Bedienpaneel angeordnet und fluchten bevorzugt mit der Bedienposition im Wesentlichen horizontal. Eine Anordnung der Bedienelemente nebeneinander auf dem Bedienpaneel kann dabei sowohl in linearer Abfolge, als auch in einer Halbkreisform oder ähnlicher leicht gebogener Anordnung stattfinden. Dass die Bedienposition nebst den Bedienelementen und den dazugehörigen Geräten im Wesentlichen horizontal fluchten soll, bedeutet, dass im Wesentlichen die Projektion eines Bedienelementes oder eines medizinischen Gerätes in eine horizontale Ebene, in der auch die Bedienposition liegt, jeweils fluchtend angeordnet ist. Höhendifferenzen in vertikaler Richtung zwischen den medizinischen Geräten und den dazugehörigen Bedienelementen oder der Bedienposition sollen hierbei nicht berücksichtigt werden.

Die Anordnung der Bedienelemente nebeneinander auf dem Bedienpaneel ermöglicht besonders gut eine modulare Ausgestaltung des Lebenserhaltungssystems, bei dem auf diese Weise mehrere medizinische Geräte und entsprechende Bedienelemente in das System eingesetzt und herausgenommen werden können.

Mit Vorteil laufen die horizontalen Fluchten der Bedienposition mit den Bedienelementen und den medizinischen Geräten der Mehrzahl radial in der Bedienposition zusammen. In dieser bevorzugten Ausführungsform sind die Bedienelemente und die entsprechenden medizinischen Geräte also radial auf die Bedienposition abgestimmt angeordnet. Alternativ ist es auch möglich, dass die Bedienelemente und entsprechenden Geräte so fluchtend angeordnet sind, dass sich die Fluchten nicht oder nicht in der Bedienposition, sondern beispielsweise in einem Bereich, der hinter der Bedienposition liegt, schneiden. Es ist auch möglich, dass die Bedienelemente und die medizinischen Geräte linear nebeneinander angeordnet sind, so dass sich deren Fluchten im Wesentlichen parallel zueinander durch den Bereich der ausgezeichneten Bedienposition verlaufen.

In der vorteilhaften Ausführungsform, in der die horizontalen Fluchten der Bedienposition mit den Bedienelementen und den medizinischen Geräten der Mehrzahl radial in der Bedienposition zusammenlaufen, ist eine besonders gute und intuitive Zuordnung der Bedienelemente zu den medizinischen Geräten gewährleistet. Dies gilt insbesondere deshalb, weil ein Bediener, der sich an der Bedienposition befindet, stets in Richtung der Flucht zwischen der Bedienposition, dem Bedienelement und dem entsprechenden medizinischen Gerät blickt.

Bevorzugt sind die Bedienelemente in einem vorgebbaren Abstandsintervall zur Bedienposition angeordnet. In einer derartigen Anordnung kann beispielsweise ein Greifbereich eines an der Bedienposition befindlichen Bedieners als Abstandsintervall vorgesehen sein.

Mit Vorteil umfasst das Lebenserhaltungssystem ferner ein Anzeigepaneel mit separaten und bevorzugt fest in das Anzeigepaneel eingebauten Anzeige- und/oder Kontrollelementen, von denen jedes Anzeige- und/oder Kontrollelement zum Anzeigen oder Kontrollieren von System und/oder Betriebszuständen eines zugeordneten medizinischen Gerätes dient. Durch die Anzeige- und/oder Kontrollelemente in dem Anzeigepaneel ist eine schnelle Überprüfung der Funktionen der medizinischen Geräte gewährleistet. Ein Bediener kann über die Anzeige- und/oder Kontrollelemente stets überprüfen, ob die medizinischen Geräte wunschgemäß funktionieren. Die Anzeige- und/oder Kontrollelemente können dabei sowohl fest in das Anzeigepaneel eingebaut als auch aus dem Anzeigepaneel herausnehmbar sein. Beispiele für Anzeige- und/oder Kontrollelemente sind Röhren- oder TFT Monitore oder Touchscreens. Die angezeigten und/oder kontrollierten Betriebszustände können dabei beispielsweise durch Pumpenzustände, Stoppuhren, Zielmarken für verschiedene Parameter der Pumpen oder des Patientenstatus, einen Batterie- und Batterieladungszustand oder durch andere für den Betrieb des Lebenserhaltungssystems relevante Daten dargestellt werden.

Besonders bevorzugt sind dabei jeweils ein Bedienelement, ein medizinisches Gerät und ein Anzeige- und/oder Kontrollelement mit der Bedienposition im Wesentlichen fluchtend angeordnet. Auf diese Weise ist es also möglich, dass sowohl das Bedienelement als auch das Anzeige- und/oder Kontrollelement mit dem entsprechenden medizinischen Gerät fluchten. Diese Anordnung ermöglicht dabei eine direkte und intuitive Zuordnung der Elemente eines medizinischen Gerätes mit dem medizinischen Gerät selber. Von der Bedienposition aus gesehen sind somit das Bedienelement, das Anzeige- und/oder Kontrollelement und das medizinische Gerät bzw. deren Projektionen in eine horizontale Ebene im Wesentlichen auf einer Geraden angeordnet. Dadurch kann ein Bediener an der Bedienposition allein durch die Position eines Bedienelementes bzw. eines Anzeige- und/oder Kontrollelementes dessen Zugehörigkeit zu einem bestimmten medizinischen Gerät des Lebenserhaltungssystems nachvollziehen. Eine übersichtliche und fehlerunanfällige Beschriftung der entsprechenden Elemente eines medizinischen Gerätes muss somit höchstens als Unterstützung der Zuordnung zwischen den einzelnen medizinischen Geräten und den entsprechenden Bedienelementen und Anzeige- und/oder Kontrollelementen vorhanden sein.

Mit Vorteil sind die Anzeige- und/oder Kontrollelemente von der Bedienposition aus gesehen jeweils unterhalb der Bedienelemente der medizinischen Geräte angeordnet. Eine derartige Anordnung stellt sicher, dass die Anzeige- und/oder Kontrollelemente von der Bedienposition aus gesehen stets leicht abgelesen werden können.

Weiter sind die Anzeige- und/oder Kontrollelemente, die medizinischen Geräte und die Bedienelemente von der Bedienposition aus gesehen mit Vorteil in, in dieser Reihenfolge, aufeinanderfolgenden Polarwinkeln angeordnet. Der Polarwinkel bezeichnet dabei konventionsgemäß einen Winkel zu einer vertikal ausgerichteten Polachse. Von einer Betrachtungsposition an der Bedienposition aus liegt also das Anzeige- und/oder Kontrollelement "über" dem medizinischen Gerät und dieses "über" dem Bedienelement, wobei "über" keine relative vertikale Position bezüglich der einzelnen Elemente bezeichnet, sondern die Neigung eines Blickbereiches bezüglich einer horizontalen Ebene beschreibt. Die Reihenfolge der genannten Anzeige- und/oder Kontrollelemente, medizinischen Geräte und der Bedienelemente kann dabei auch umgekehrt sein, so dass das Anzeige- und/oder Kontrollelement "unter" den medizinischen Geräten und diese "unter" den Bedienelementen von der Bedienposition aus gesehen, angeordnet sind. Die unterschiedlichen Polarwinkel, in denen die Anzeige- und/oder Kontrollelemente, die medizinischen Geräte und die Bedienelemente von der Bedienposition aus gesehen, angeordnet sind, können dabei sowohl durch eine tatsächliche vertikale Beabstandung als auch eine horizontale Beabstandung in unterschiedlichen Abständen von der Bedienposition aus zustande kommen. Diese Anordnung kann also auch dann erfüllt sein, wenn die Bedienelemente von der Bedienposition aus gesehen vor den medizinischen Geräten und diese unterhalb der Anzeige- und/oder Kontrollelemente jeweils in horizontaler Flucht mit der Bedienposition angeordnet sind.

Besonders bevorzugt sind jeweils ein Bedienelement, ein medizinisches Gerät und ein Anzeige- und/oder Kontrollelement auf einem Ergonomieradius angeordnet. Die Anordnung auf einem Ergonomieradius stellt dabei sicher, dass das Bedienelement, das medizinische Gerät und das Anzeige- und/oder Kontrolleelement jeweils von der Bedienposition aus erreichbar ist. Dabei wird in Abhängigkeit einer vertikalen Höhe in Bezug auf die Bedienposition ein horizontaler Abstand von der Bedienposition angenommen, den ein Benutzer an der Bedienposition in der entsprechenden Höhe erreicht. Der horizontale Abstand ist dabei in einer Armhöhe eines Bedieners an der Bedienposition am größten, und nimmt nach oben sowie nach unten zunehmend ab.

Besonders bevorzugt ist das Lebenserhaltungssystem dadurch gekennzeichnet, dass das Bedienpaneel oberhalb der Kniehöhe eines in der Bedienposition sitzenden Bedieners über die Grundfläche der Mehrzahl medizinischer Geräte auskragt, dass ferner der Abstand der Bedienelemente zur Bedienposition in einem Greifbereich des Bedieners liegt und die Anzeige- und/oder Kontrollelemente in einer Augenhöhe des Bedieners positionierbar sind. Durch eine derartige Ausgestaltung des Lebenserhaltungssystems kann sichergestellt werden, dass eine intuitive sowie ergonomisch angenehme Bedienung des Lebenserhaltungssystems möglich ist. Ein in der Bedienposition sitzender Bediener, dessen Kniehöhe ungeachtet der Körpergröße im Wesentlichen konstant ist und oberhalb derer das Bedienpaneel über die Grundfläche der Mehrzahl der medizinischen Geräte auskragt, kann somit die Bedienelemente auf dem Bedienpaneel sitzend bequem erreichen und die medizinischen Geräte, die fluchtend mit den Bedienelementen angeordnet sind, intuitiv bedienen. Dass der Abstand der Bedienelemente zur Bedienposition in einem Greifbereich des Bedieners liegt, macht die Bedienung der Bedienelemente zudem angenehmer. Auch das Ablesen der Anzeige- und/oder Kontrollelemente, die in einer Augenhöhe des Bedieners positionierbar sind, ist in der Augenhöhe des Bedieners besonders angenehm. Somit wird also ein Lebenserhaltungssystem zur Verfügung gestellt, das eine intuitive und dadurch besonders fehlerfreie Bedienung eines Lebenserhaltungssystems bei gleichzeitiger ergonomischer Ausgestaltung dieses Lebenserhaltungssystems ermöglicht wird. Durch die ergonomische Ausgestaltung des Lebenserhaltungssystems ist neben der fehlerfreien Bedienbarkeit durch die intuitive Anordnung der Gerätschaften zudem ein hoher arbeitsmedizinischer Standard ermöglicht.

Bevorzugt ist mindestens eines der Seitenelemente flächig ausgedehnt. Durch eine derartige Ausführung der Seitenelemente können diese nicht nur besonders stabil ausgeführt sein, sondern bilden auch in seitlicher Richtung einen guten Schutz für die zwischen den Seitenelementen aufgenommenen Teile des Lebenserhaltungssystems. Alternativ kann auch eines oder mehrere der Seitenelemente stabförmig ausgebildet sein.

In einer weiteren bevorzugten Ausführungsform sind die Seitenelemente auf Rollen gelagert und vertikal auf sie wirkende Kraftkomponenten werden über die Rollen abgetragen. Damit wird das Lebenserhaltungssystem in die Lage versetzt, beispielsweise in einem Krankenhaus zwischen verschiedenen Operationssälen, zu einzelnen Patienten in einer Intensivstation oder auf Patientenzimmern bewegt zu werden. Die Seitenelemente sind dabei derart gestaltet, dass die vertikal auf sie wirkenden Kraftkomponenten über die Rollen abgetragen werden. Dabei muss nicht jedes der Seitenelemente auf Rollen gelagert sein. Es ist beispielsweise auch möglich, dass bei zwei Seitenelementen nur ein Seitenelement auf Rollen gelagert und das andere nicht rollfähig ausgeführt ist. In diesem Fall kann beispielsweise das nicht rollfähige Seitenelement angehoben und das Lebenserhaltungssystem auf den Rollen des verbleibenden Seitenelements verschoben werden. Die Kraftabtragung über die Seitenelemente macht das Lebenserhaltungssystem in Verbindung mit der Ausbildung eines Rahmens besonders als mobilen Gerätekomplex tauglich.

Bevorzugt ist durch die Seitenelemente und das Verbindungselement ein im Wesentlichen abgeschlossener Raum definiert. Dies bedeutet, dass die Seitenelemente und das Verbindungselement Begrenzungsflächen dieses abgeschlossenen Raums darstellen können. Zwischen den Seitenelementen und dem Verbindungselement, also in dem im Wesentlichen abgeschlossenen Raum, können dabei unterschiedliche medizinische Geräte, Stromversorgungseinrichtungen, wie beispielsweise eine Batterie, Elektronikelemente oder Kabel und/oder Schlauchverbindungen untergebracht werden, die somit als der Mobilität nicht im Wege stehen.

Mit Vorteil ist dabei zwischen zwei der Seitenelemente eine im Wesentlichen flächig und vertikal ausgedehnte, u. U. aus Blech gefertigte Abdeckung angebracht. Die Abdeckung bildet also beispielsweise vertikale Wände. Dabei bietet die Abdeckung Schutz für die zwischen den Seitenelementen untergebrachten Teile der medizinischen Geräte und lenkt dabei das Augenmerk des Bedieners auf die wesentlichen Bedienelemente, indem zum Betrieb notwendige, aber für die Bedienung unerhebliche Teile, wie beispielsweise eine Batterie, Steckplätze für Leiterplatten oder Kabel und Schlauchverbindungen hinter der Abdeckung und damit außerhalb des Sichtbereiches eines Bedieners oder eines Patienten untergebracht sind. Die Abdeckung kann dabei aus Blech sowie aus anderen Materialien gefertigt sein.

Mit Vorteil umschließt die Abdeckung den Raum in Richtung einer horizontalen Ebene und weist bevorzugt eine öffnungsfähige Klappe auf. In einer derartigen Ausgestaltung des Lebenserhaltungssystems wird der Raum zwischen den Seitenelementen durch die Abdeckung, welche beispielsweise zwei Blechplatten an einer Vorder- und einer Rückseite zwischen den beispielsweise flächig ausgebildeten Seitenelementen, ein weitgehend geschlossenes Volumen erzeugt werden. Eine obere Begrenzung dieses Volumens zwischen den Seitenelementen und der Abdeckung kann dabei zwar ebenfalls durch eine solche Abdeckung abgedeckt sein, wird jedoch bevorzugt beispielsweise durch ein oder mehrere Verbindungselemente sowie die medizinischen Geräte und/oder deren Bedienelemente eingenommen. Eine untere Begrenzung des Volumens kann durch eine weitere Abdeckung vorhanden sein, das Volumen kann aber auch nach unten, beispielsweise zu Kühlzwecken, offen bleiben. Durch das Vorhandensein einer öffnungsfähigen Klappe, die beispielsweise zu einer Vorder- und/oder Rückseite des Lebenserhaltungssystems weisen kann, ist es möglich, an die in den Volumen zwischen den Seitenelementen und der Abdeckung befindlichen Teile der medizinischen Geräte, wie beispielsweise Leiterplatten, Kabel und/oder Schlauchverbindungen oder eine darin befindliche Stromversorgung von außen zu gelangen. Somit ist eine besonders leichte Wartbarkeit des Lebenserhaltungssystems gewährleistet. Die in den Volumen untergebrachten medizinischen oder Peripheriegeräte sind somit im täglichen Betrieb abgedeckt und dennoch beispielsweise zu Wartungszwecken leicht zugänglich.

Bevorzugt weist das Lebenserhaltungssystem in dem Raum ein Anschlusselement zum Anschließen des medizinischen Gerätes oder der medizinischen Geräte auf, wobei das Anschlusselement durch ein Seitenelement gehalten ist und bevorzugt eine Vielzahl von Steckplätzen für elektronische Steckplatten aufweist. Der Raum zwischen den Seitenelementen eignet sich aufgrund seiner zentralen Lage besonders gut dafür, wichtige Elektronikelemente zentral dort unterzubringen. Darüber hinaus ist der Raum zwischen den Seitenelementen, beispielsweise durch eine Abdeckung, abschließbar, so dass die dort vorhandenen Elektronikelemente sowohl vor äußeren Einwirkungen geschützt als auch verborgen sind.

Bevorzugt sind die Seitenelemente und das Verbindungselement aus Aluminiumsandguss, insbesondere einstückig gefertigt. Die Seitenelemente und das Verbindungselement, das auch zwei oder mehr Verbindungen zwischen den Seitenelementen umfassen kann, bilden dabei einen Torsionskasten, der die Festigkeit des Lebenserhaltungssystems sicherstellt.

Der oben beschriebene Aufbau des Lebenserhaltungssystems, d.h. der Herz-Lungen-Maschine ermöglicht es, dass Kabelverbindungen zwischen einzelnen medizinischen Geräten, die im oberen Bereich des Lebenserhaltungssystems angeordnet sind, oben in dem Torsionskasten verlaufen können. Durch die oben verlaufenden Kabelverbindungen und die Kastenform des Lebenserhaltungssystems wird daher ein guter Zugang für Wartungsarbeiten, Konfiguration und Batterieaustausch ermöglicht. Das beschriebene Lebenserhaltungssystem ist darüber hinaus bevorzugt durch ein Masterweiterungssystem erweiterbar. Die Seitenteile können neben einer einstückigen Ausführung mit dem Verbindungsteil auch durch das Verbindungsteil miteinander verschraubt sein. Die Seitenteile bilden bei dem beschriebenen Gerätekomplex Halterungen, zwischen denen sich ein großes Volumen, beispielsweise zum Unterbringen der Elektronik, beispielsweise einer Platine und Steckkarten, wie oben beschrieben, befindet. Da die Stabilität des Gerätekomplexes durch die Seitenteile und das dazwischen befindliche Verbindungsteil sichergestellt wird, ist es zudem möglich, dass zwischen den Seitenteilen beispielsweise auch ein Raum für die Knie eines an einer Bedienposition sitzenden Bedieners vorgesehen ist. Die Platine und Steckkarten sind dabei im Volumen zwischen den Seitenteilen bevorzugt schräg eingebaut, um Platz für die Knie zu schaffen. Es ist auch möglich, dass die medizinischen oder Peripheriegeräte im oben beschriebenen Gerätekomplex modular einsetzbar und austauschbar sind. Dies kann insbesondere auch durch ein Stecksystem der elektronischen Geräte, durch das ein richtiger Anschluss der entsprechenden elektrischen und elektronischen Kontakte sichergestellt wird, realisiert werden. Da sich die Seitenelemente als Halterungen eignen, sind keine weiteren Ausleger für einen sicheren Halt des Lebenserhaltungssystems nötig. Insbesondere können vertikale Kräfte über die Seitenelemente direkt abgetragen werden.

Hinsichtlich des erfindungsgemäßen Lebenserhaltungssystems kann ein Anzeige- und/oder Kontrollelement in seiner Höhe variabel sein. Dies kann insbesondere dadurch ermöglicht werden, dass das Anzeige- und/oder Kontrollelement an einem oder mehreren vertikal ausgerichteten Masten befestigt ist. Mit Vorteil sind die Anzeige- und/oder Kontrollelemente dabeium eine horizontale Achse so gekippt, dass ein Bediener, der an der Bedienposition sitzt, angenehm auf diese Elemente blicken und diese ablesen kann. Da die Unterschenkelhöhe eines erwachsenen Bedieners weitgehend invariabel ist, kann die Höhe des Bedienpaneels, das im Wesentlichen flächig über eine Grundfläche der Mehrzahl medizinischer Geräte auskragend ausgebildet ist, fest ausgeführt sein. Dies erleichtert den konstruktiven Aufbau des Lebenserhaltungssystems und verringert die Produktionskosten. Bevorzugt kann zusätzlich die Entfernung zwischen dem Auge eines Bedieners und dem Anzeige- und/oder Kontrollelement angepasst werden, wegen der Unterfahrbarkeit des im Wesentlichen flächigen Bedienpaneels ist zudem die Entfernung zwischen dem Rumpf des Bedieners und den Bedienelementen der medizinischen Geräte anpassbar. Die Höhe des Bedienpaneels entspricht bevorzugt einer Ellenbogenhöhe eines sitzenden Bedieners und die Breite des Bedienpaneels ist bevorzugt begrenzt. Eine bevorzugte maximale Breite des Bedienpaneels ist dabei 70cm. Für eine ergonomische Positionierung, insbesondere für eine korrekte Sitzhöhe und Rumpfneigung, des Bedieners eignet sich insbesondere ein ergonomischer Stuhl.

Weitere vorteilhafte Ausführungsformen werden durch die nachfolgende Detailbeschreibung der Figuren sowie die Patentansprüche beschrieben.

### Kurze Figurenbeschreibung

- Fig. 1: zeigt eine Draufsicht auf ein Lebenserhaltungssystem;
- Fig. 2: zeigt eine Seitenansicht des Lebenserhaltungssystems aus Fig. 1;
- Fig. 3: zeigt eine Vorderansicht des Lebenserhaltungssystems aus Figuren 1 und 2;
- Fig. 4: zeigt einen medizinischen Gerätekomplex in einer perspektivischen Vorderansicht;
- Fig. 5: ist eine perspektivische Schnittansicht des medizinischen Gerätekomplexes aus Fig. 4; und
- Fig. 6: zeigt eine Seitenansicht des medizinischen Gerätekomplexes aus Figuren 4 und 5 in einer Schnittdarstellung.

### Bevorzugte Ausführungsformen

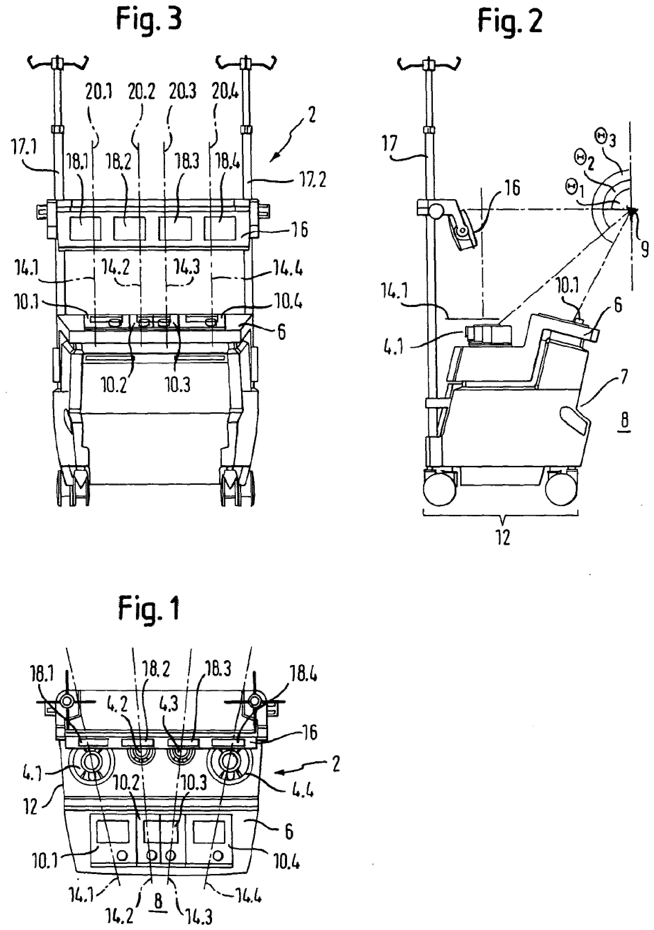

Fig. 1 zeigt ein Lebenserhaltungssystem, nämlich eine Herz-Lungen-Maschine 2, in einer Draufsicht. In dem unteren Teil der Figur 1 sind vier Bedienelemente 10.1 bis 10.4 dargestellt, die sich linear nebeneinander angeordnet auf einem Bedienpaneel 6 der Herz-Lungen-Maschine 2 befinden. Jedes der Bedienelemente 10.1 bis 10.4 umfasst dabei einen Drehregler und ein Eingabe- und/oder Anzeigeelement.

An das Bedienpaneel 6 mit den Bedienelementen 10.1 bis 10.4 schließt sich oberhalb ein Bereich an, in dem vier medizinische Pumpen 4.1 bis 4.4 angeordnet sind. Die medizinischen Pumpen 4.1 bis 4.4 bilden dabei eine Mehrzahl medizinischer Geräte und sind ebenfalls im Wesentlichen linear nebeneinander angeordnet. Dabei definiert die Anordnung der Pumpen 4.1 bis 4.4 sowie deren notwendige Peripheriegeräte wie beispielsweise elektronische Steuerungen oder eine Batterie eine Grundfläche 12, in der die Mehrzahl medizinischer Gerätschaften angeordnet ist. In Fig. 1 ist deutlich zu erkennen, dass das Bedienpaneel 6 mit den Bedienelementen 10.1 bis 10.4 flächig über die Grundfläche 12, in Richtung des unteren Bildrands von Fig. 1, hinausragt.

Es ist zu erkennen, dass Fluchten 14.1 bis 14.4, auf denen je eine Pumpe 4.1 bis 4.4 und ein dazugehöriges Bedienelement 10.1 bis 10.4 liegen, in einer ausgezeichneten Bedienposition 8 zusammenlaufen. Das Zusammenlaufen der Fluchten 14.1 bis 14.4 zeichnet die Bedienposition 8 dabei gegenüber anderen möglichen Positionen, von denen aus das medizinische Gerät auch bedienbar ist, aus. Fig. 1 zeigt dabei deutlich, wie die jeweiligen Fluchten 14.1 bis 14.4 zwischen den Pumpen 4.1 bis 4.4 und den dazugehörigen Bedienelementen 10.1 bis 10.4 in einer Bedienposition 8 fächerförmig zusammenlaufen. An der ausgezeichneten Bedienposition 8, in der die Fluchten 14.1 bis 14.4. zusammenlaufen, hält sich ein Bediener des medizinischen Gerätes während des Bedienens des medizinischen Geräts bevorzugt auf.

Des Weiteren ist in Figur 1 ein Anzeigepaneel 16 im oberen Bildbereich zu sehen, in dem vier Monitore 18.1 bis 18.4 eingelassen sind. Die Monitore 18.1 bis 18.4 liegen dabei ebenfalls auf den jeweiligen Fluchten 14.1 bis 14.4. Es liegt also sowohl ein Bedienelement 10.1 bis 10.4, die dazugehörige Pumpe 4.1 bis 4.4 als auch der dazugehörige Monitor 18.1 bis 18.4 auf je einer Flucht 14.1 bis 14.4.

Fig. 2 zeigt eine Seitenansicht der Herz-Lungen-Maschine 2 aus Fig. 1. Neben den schon in Fig. 1 beschriebenen Elementen zeigt Fig. 2 ein Mastsystem 17, an dem das Anzeigepaneel 16 befestigt ist. Das Anzeigepaneel 16 befindet sich dabei im Wesentlichen vertikal oberhalb der Pumpen 4.1 bis 4.4, von denen in Fig. 2 lediglich die Pumpe 4.1 zu sehen ist, und ist in seiner Höhe entlang des Mastsystems 17 stufenlos einstellbar. Die Einstellung der Höhe des Anzeigepaneels 16 richtet sich dabei nach den Körpermaßen eines an der Bedienposition 8 befindlichen Bedieners.

Fig. 2 zeigt dabei deutlich, dass das Bedienpaneel 6 flächig über die Grundfläche 12 der Mehrzahl medizinischer Geräte 4.1 bis 4.4 auskragend ausgebildet ist. Weiter ist in Fig. 2 zu erkennen, dass das Anzeigepaneel 16, die Pumpe 4.1 und das Bedienelement 10.1 auf dem Bedienpaneel 6 von einer Blickposition 9 eines an der Bedienposition 8 befindlichen Bedieners aus gesehen in aufeinanderfolgenden Polarwinkeln θ₁ bis θ₃ angeordnet sind. Dabei liegt das Anzeigepaneel 16 im Wesentlichen auf derselben Höhe wie der Betrachtungspunkt 9. Der Polarwinkel θ₁ zwischen der Blickrichtung vom Betrachtungspunkt 9 zum Anzeigepaneel 16 und der vertikal nach oben zeigenden Polarachse beträgt hier also etwa 90°. Darunter, in einem etwas größeren Polarwinkel θ₂ befindet sich die Pumpe 4.1, an die sich in einem noch größeren Polarwinkel θ₃ das auf dem Bedienpaneel 6 befindliche Bedienelement 10.1 anschließt. Fig. 2 zeigt dabei, dass die eigentliche Höhe des Bedienelements 10.1 über der Höhe der Pumpe 4.1 liegt. Trotzdem ist der Polarwinkel θ₃, unter dem ein Betrachter am Betrachtungspunkt 9 auf das Bedienelement 10.1 blickt, größer als der Polarwinkel θ₂, unter dem der Betrachter von der Betrachtungsposition 9 aus die Pumpe 4.1 betrachtet. Die Pumpe 4.1 ist dabei von der Bedienposition 8 aus gesehen hinter dem Bedienelement 10.1 angeordnet.

Die soeben beschriebenen Zusammenhänge zwischen dem Bedienelement 10.1, der Pumpe 4.1 und dem Anzeigepaneel 16, in dem sich unter anderem der Monitor 18.1 befindet, gelten analog auch für die übrigen Bedienelemente 10.2 bis 10.4, die Pumpen 4.2 bis 4.4 und das Anzeigepaneel 16 mit den übrigen Monitoren 18.2 bis 18.4.

Die Seitenansicht in Fig. 2 zeigt zudem eine Aussparung 7, welche die Knie eines an der Bedienposition 8 sitzenden Bedieners aufnehmen kann, so dass der Bediener an der Bedienposition 8 sowohl sitzen, als auch sich nahe an den Bedienelementen 10.1 bis 10.4 befinden kann.

Fig. 3 zeigt eine Vorderansicht der in den Figuren 1 und 2 dargestellten Herz-Lungen-Maschine 2. Im mittleren Bereich der Abbildung sind die Bedienelemente 10.1 bis 10.4 auf dem Bedienpaneel 6 angeordnet dargestellt. Die Fluchten 14.1 bis 14.4 verlaufen dabei ausgehend von der Bedienposition 8, deren Perspektive die Fig. 3 weitgehend wiedergibt, über die Bedienelemente 10.1 bis 10.4, die Pumpen 4.1 bis 4.4 (nicht dargestellt) und Monitore 18.1 bis 18.4 in dem Anzeigepaneel 16 als Anzeige- und/oder Kontrollelemente, die in der in Fig. 3 dargestellten Ausführungsform beispielsweise durch Touch-Screen-Monitore realisiert werden. Die Vorderansicht der Herz-Lungen-Maschine 2 zeigt, dass das Mastsystem 17 aus Fig. 2 durch zwei Masten 17.1, 17.2 gebildet wird, welches das Anzeigepaneel 16 und damit die Monitore 18.1 bis 18.4 in ihrer vertikalen Position stufenlos einstellbar befestigt.

Darüber hinaus ist in Fig. 3 angedeutet, dass die Bedienelemente 10.1 bis 10.4, die Pumpen 4.1 bis 4.4 (nicht dargestellt) und die Monitore 18.1 bis 18.4 auf Ergonomieradien 20.1 bis 20.4 liegen, wobei jeder Ergonomieradius 20.1 bis 20.4 zusammen mit der jeweiligen Flucht 14.1 bis 14.4 in jeweils einer vertikalen Ebene liegt. Es ist also zu sehen, dass die Bedienelemente 10.1 bis 10.4, die Pumpen 4.1 bis 4.4 (nicht dargestellt) und die Monitore 18.1 bis 18.4 auf je einer Ebene liegen, in der die entsprechende Flucht 14.1 bis 14.4 und der entsprechende, im Wesentlichen vertikal ausgerichtete, Ergonomieradius 20.1 bis 20.4 liegen.

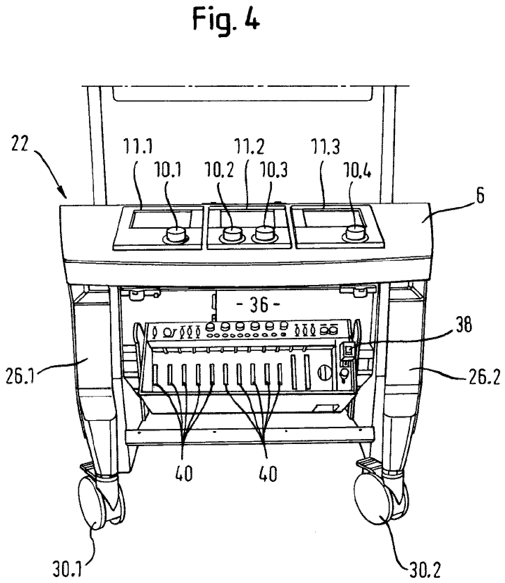

Fig. 4 zeigt einen medizinischen Gerätekomplex 22, der in diesem Fall ebenfalls eine Herz-Lungen-Maschine bildet. Der medizinische Gerätekomplex 22 umfasst dabei zwei Seitenteile 26.1 und 26.2, die flächig und horizontal beabstandet, einen Raum 36 zwischen sich definieren. Die Seitenelemente 26.1, 26.2 sind dabei auf vier Rollen gelagert, von denen in Fig. 4 nur die Rollen 30.1 und 30.2 der Vorderseite des medizinischen Gerätekomplexes zu sehen sind. In Fig. 4 nicht dargestellt ist ein Verbindungselement 28, welches die zwei Seitenteile 26.1, 26.2 voneinander beabstandet und diese formstabil miteinander verbindet. Das Verbindungselement 28 sowie die Seitenteile 26.1, 26.2 sind dabei in der hier beschriebenen bevorzugten Ausführungsform aus Aluminiumsandguss gefertigt und können auch einstückig als Torsionsrahmen ausgeführt sein. Dabei ist das Verbindungselement 28 in der hier dargestellten Ausführungsform zwar nur an einer oberen Kante der Seitenelemente 26.1, 26.2 vorgesehen, es kann aber auch ein weiteres Verbindungselement vorgesehen sein, das die unteren Kanten der Seitenelemente 26.1, 26.2 miteinander verbindet. In diesem Fall entsteht ein geschlossener Torsionsrahmen, der sich durch eine besonders hohe Festigkeit auszeichnet.

In dem Raum 36 zwischen den Seitenelementen 26.1, 26.2 ist eine Steckvorrichtung 38 untergebracht, die eine Vielzahl von Steckplätzen 40 für Steckplatten oder dergleichen aufweist. Die Steckvorrichtung 38 ist dabei schräg bezüglich einer horizontalen Ebene im Raum 36 zwischen den Seitenelementen 26.1, 26.2 eingebaut, um ein leichtes Einstecken von Steckplatten in die Steckplätze 40 zu ermöglichen und den Raum 36 zwischen den Seitenelementen 26.1, 26.2 dabei effizient zu nutzen.

Oberhalb des Raums 36 ist in Fig. 4, analog zu Fig. 1 bis 3 ein Bedienpaneel 6 gezeigt, das vier Bedienelemente 10.1 bis 10.4 umfasst. Die vier Bedienelemente 10.1 bis 10.4 sind in drei Bedienmodulen 11.1 bis 11.3 untergebracht. Dabei umfasst das mittlere Bedienmodul 11.2 zwei Bedienelemente 10.2 und 10.3, was eine platzsparende Anordnung der Bedienelemente 10.1 bis 10.4 erleichtert. Die Bedienelemente 10.1 bis 10.4 können jedoch auch als jeweils einzelne Module in das Bedienpaneel 6 eingesetzt werden. Jedes der Bedienmodule 11.1 bis 11.3 ist dabei in das Bedienpaneel 6 eingelassen und somit ist es leicht möglich, dass ein oder mehrere Bedienmodule 11.1 bis 11.3 und damit die entsprechenden Bedienelemente 10.1 bis 10.4 aus dem Bedienpaneel 6 herausgenommen oder ersetzt werden. Dies führt zu einer besonders einfachen Möglichkeit, den Gerätekomplex umzugestalten und verringert zudem den Aufwand für mögliche Wartungsarbeiten, da einzelne Module ersetzbar sind, das Gerät somit sofort wieder einsatzbereit ist und das zu wartende oder zu reparierende Modul an einem dafür vorgesehenen Ort unter Verwendung umfangreicherer technischer Möglichkeiten bearbeitet werden kann. Es lassen sich natürlich auch andere Module als die hier gezeigten Bedienmodule 11.1 bis 11.3 in das Bedienpaneel 6 einsetzen.

Die Anschlüsse der Bedienelemente 10.1 bis 10.4 und übriger in dem medizinischen Gerätekomplex 22 enthaltener Geräte sind dabei bevorzugt vom Raum 36 aus erreichbar, wo sie in einem oberen Bereich zwischen den Seitenelementen 26.1, 26.2 durch Kabel und/oder Schlauchverbindungen anschließbar sind. Die Kabel und/oder Schlauchverbindungen (nicht gezeigt) verlaufen dabei bevorzugt in einem oberen Bereich des Raums 36 und werden gegebenenfalls mit der Steckvorrichtung 38 verbunden.

Die für die Bedienelemente 10.1 bis 10.4 und deren Module 11.1 bis 11.3 genannten Eigenschaften gelten analog auch für die im medizinischen Gerätekomplex 22 vorhandenen medizinischen Geräte, wie beispielsweise Pumpen. Insgesamt ist der hier beschriebene medizinische Gerätekomplex 22 modular aufgebaut, sodass nahezu uneingeschränkt einzelne Module auf dem Gerätekomplex 22 vorhanden sein und durch einander ausgetauscht werden können.

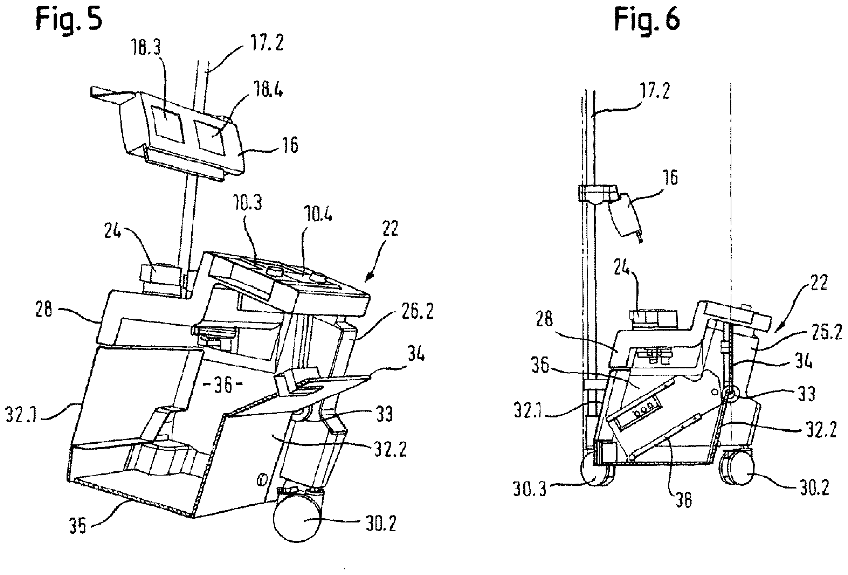

Fig. 5 zeigt eine dreidimensionale Schnittdarstellung des medizinischen Gerätekomplexes 22, wie er in Fig. 4 beschrieben wurde. Neben den bereits beschriebenen Elementen zeigt Fig. 5 eine vordere und eine hintere Abdeckung 32.1, 32.2, wobei die vordere Abdeckung 32.2 ferner eine öffnungsfähige Klappe 34 aufweist, die in Fig. 5 in der geöffneten Position dargestellt ist.

Die öffnungsfähige Klappe 34 lässt sich dabei um eine horizontal ausgerichtete Drehachse, nämlich um ein am unteren Rand der Klappe 34 angeordnetes Gelenk 33 nach unten aufklappen. Die öffnungsfähige Klappe 34 bietet den Vorteil, dass sie in geöffnetem Zustand einen leichten Zugang zum Inneren des zwischen den Seitenelementen 26.1, 26.2 und dem Verbindungselement 28 zwischen den Seitenelementen 26.1, 26.2 vorhandenen Raum 36 freigibt.

In dem Raum 36 kann die schon in Fig. 4 dargestellte Steckvorrichtung 38 sowie weitere Peripheriegeräte, Elektronik oder eine Batterie zur Stromversorgung untergebracht werden. Bei geschlossener Klappe 34 sind diese Elemente des Gerätekomplexes 22 durch die Seitenelemente 26.1, 26.2, die Abdeckung 32.1, 32.2, das Verbindungselement 28 und ein Bodenteil 35 geschützt in dem Raum 36 aufgenommen und können trotzdem durch einfaches Öffnen der Klappe 34 schnell und bequem erreicht werden. Dies ist insbesondere zu Wartungszwecken von besonderem Vorteil.

Ein wichtiges und relativ oft zu wartendes Element eines mobilen medizinischen Gerätekomplexes 22 ist die bevorzugt wieder aufladbare Batterie, mit der in den allermeisten Fällen die Stromversorgung der medizinischen Gerätschaften des Komplexes sichergestellt wird. Die Kapazität der Batterie nimmt mit zunehmender Lebensdauer ab und kann somit zu einem bedeutenden Risikofaktor werden. Um hier vorzubeugen wird die Batterie schon bei ersten Anzeichen einer Alterung oder auch in regelmäßigen Abständen überprüft und gegebenenfalls ausgetauscht. Um diesen Wartungsschritt sowie andere Wartungsarbeiten wie etwa ein Überprüfen von Schlauchleitungen oder Austauschen elektronischer Bauteile möglichst einfach und schnell zu ermöglichen, bietet die öffnungsfähige Klappe 34 einen montagefreien Zugang zu dem Raum 36 im Innern des Torsionsrahmens aus den Seitenteilen 26.1, 26.2 und der Verbindung 28.

Fig. 5 zeigt eine Ansicht des Gerätekomplexes 22, bei dem neben den Bedienelementen 10.3, 10.4 ein medizinisches Gerät 24 in Form einer Pumpe zu sehen ist. Die Pumpe ist wie bereits oben beschrieben modular in den Gerätekomplex 22 einsetzbar, austauschbar und auch entfernbar. Darüber befinden sich analog zu den vorher beschriebenen Figuren zwei Monitore 18.3, 18.4, die in einem Anzeigepaneel 16 eingelassen sind. Das Anzeigepaneel 16 wiederum ist an einem Mast 17.2, der Teil eines Mastsystems 17 ist, befestigt.

Fig. 6 zeigt den medizinischen Gerätekomplex, der schon in Fig. 4 und Fig. 5 dargestellt wurde, in einer Seitenansicht. Die in Fig. 6 dargestellte Seitenansicht ist zudem eine Schnittdarstellung des medizinischen Gerätekomplexes 22. Die Fig. 6 zeigt das Verbindungselement 28, das sich zwischen den Seitenelementen 26.1, 26.2 erstreckt und diese formstabil miteinander verbindet. Das Verbindungselement 28 sowie die Seitenelemente 26.1, 26.2 und gegebenenfalls ein weiteres Verbindungselement, das beispielsweise an einem unteren Rand der Seitenelemente 26.1, 26.2 befestigt sein könnte, können dabei auch einstückig ausgeführt sein.

Das Verbindungselement 28 dient, wie in Fig. 6 gut zu erkennen ist, gleichzeitig der Aufnahme eines oder mehrerer medizinischer Geräte, von denen hier nur ein medizinisches Gerät 24 dargestellt ist, das wie schon in den vorher beschriebenen Figuren durch eine medizinische Pumpe gebildet wird.

Neben dem in Fig. 6 dargestellten Raum 36 und der darin angeordneten Steckvorrichtung 38, die wie beschrieben schräg eingebaut ist, zeigt Fig. 6 die vordere und die hintere Abdeckung 32.1, 32.2, wobei die vordere Abdeckung 32.2 ferner die öffnungsfähige Klappe 34 aufweist. Die Klappe 34 lässt sich wie in Fig. 5 dargestellt um das Gelenk 33 aufklappen, ist in Fig. 6 jedoch in ihrer geschlossenen Position dargestellt. Zusätzlich zu Fig. 4 und Fig. 5 zeigt Fig. 6 eine weitere Rolle 30.3, auf der das Seitenelement 26.2 gelagert ist. Analog dazu ist auch das Seitenelement 26.1 auf zwei Rollen gelagert.

## Patentansprüche

1. Herz-Lungen-Maschine (2), mit
- mehreren medizinischen Geräten (4.1-4.4, 24),
- einem Bedienpaneel (6),
- zwei oder mehr sich im Wesentlichen vertikal erstreckenden Seitenelementen (26.1, 26.2) und
- einem sich zwischen den Seitenelementen (26.1, 26.2) und bevorzugt im Wesentlichen horizontal erstreckenden Verbindungselement (28);
- wobei das Bedienpaneel (6) separate Bedienelemente (10.1-10.4) zum Bedienen der medizinischen Geräte (4.1-4.4, 24) aufweist;
- wobei das Verbindungselement (28) die Seitenelemente (26.1, 26.2) miteinander verbindet,
- wobei das Verbindungselement (28) und die Seitenelemente (26.1, 26.2) einen Rahmen zum Tragen der medizinischen Geräte (4.1-4.4, 24) bilden, so dass die medizinischen Geräte (4.1-4.4, 24) zumindest teilweise zwischen den Seitenelementen (26.1, 26.2) aufnehmbar sind, und
- wobei das Bedienpaneel (6) über eine Grundfläche (12) der medizinischen Geräte (4.1-4.4, 24) auskragend ausgebildet ist,
**dadurch gekennzeichnet, dass**
- eine ausgezeichnete Bedienposition (8) dadurch ausgezeichnet ist, dass das Bedienpaneel (6) derart ausgebildet ist, dass jedem Gerät (4.1-4.4, 24) ein mit nur diesem Gerät (4.1-4.4, 24) und mit der Bedienposition (8) fluchtendes Bedienelement (10.1-10.4) zugeordnet ist.

2. Herz-Lungen-Maschine (2) nach Anspruch 1, **dadurch gekennzeichnet, dass** mindestens eines der Seitenelemente (26.1, 26.2) flächig ausgedehnt ist.

3. Herz-Lungen-Maschine (2)nach Anspruch 1 oder 2, **dadurch gekennzeichnet, dass** die Seitenelemente (26.1, 26.2) auf Rollen (30.1-30.3) gelagert sind und vertikal auf sie wirkende Kraftkomponenten über die Rollen (30.1-30.3) abtragen.

4. Herz-Lungen-Maschine (2)nach einem der Ansprüche 1 bis 3, **dadurch gekennzeichnet, dass** durch die Seitenelemente (26.1, 26.2) und das Verbindungselement (28) ein im Wesentlichen abgeschlossener Raum (36) definiert ist.

5. Herz-Lungen-Maschine (2)nach einem der Ansprüche 1 bis 4, **dadurch gekennzeichnet, dass** zwischen zwei der Seitenelemente (26.1, 26.2) eine im Wesentlichen flächig und vertikal ausgedehnte, bevorzugt aus Blech gefertigte Abdeckung (32.1, 32.2) angebracht ist.

6. Herz-Lungen-Maschine (2)nach Anspruch 4 und 5, **dadurch gekennzeichnet, dass** die Abdeckung (32.1, 32.2) den Raum (36) in Richtung einer horizontalen Ebene umschließt und bevorzugt eine öffnungsfähige Klappe (34) aufweist.

7. Herz-Lungen-Maschine (2)nach einem der Ansprüche 4 bis 6, **dadurch gekennzeichnet, dass** er in dem Raum (36) ein Anschlusselement (38) zum Anschließen des medizinischen Geräts (24) oder der medizinischen Geräte aufweist, wobei das Anschlusselement (38) durch ein Seitenelement (26.1, 26.2) gehalten ist und bevorzugt eine Vielzahl von Steckplätzen (40) für elektronische Steckplatten aufweist.

8. Herz-Lungen-Maschine (2)nach einem der Ansprüche 1 bis 7, **dadurch gekennzeichnet, dass** eines oder mehrere der Seitenelemente (26.1, 26.2) stabförmig ausgebildet ist bzw. sind.

## Claims

1. Heart-lung machine (2), comprising
- a plurality of medical devices (4.1-4.4, 24),
- an operating panel (6),
- two or more side elements (26.1, 26.2) substantially extending vertically and
- a connecting element (28) extending between the side elements (26.1, 26.2) and preferably extending substantially horizontally;
- wherein the operating panel (6) comprises separate operating elements (10.1-10.4) for operating the medical devices (4.1-4.4, 24);
- wherein the connecting element (28) connects the side elements (26.1, 26.2) to one another,
- wherein the connecting element (28) and the side elements (26.1, 26.2) form a frame for carrying the medical devices (4.1-4.4, 24), such that the medical devices (4.1-4.4, 24) can be at least partially received between the side elements (26.1, 26.2), and
- wherein the operating panel (6) is formed so as to project over a base surface (12) of the medical devices (4.1-4.4, 24),
**characterised in that**
- an outstanding operating position (8) is distinguished **in that** the operating panel (6) is designed such that to each device (4.1-4.4, 24) is assigned an operating element (10.1-10.4) aligned with only that one of the devices (4.1-4.4, 24) and with the operating position (8).

2. Heart-lung machine (2) according to claim 1, **characterised in that** at least one of the side elements (26.1, 26.2) is planarly extending.

3. Heart-lung machine (2) according to claim 1 or 2, **characterised in that** the side elements (26.1, 26.2) are mounted on rolls (30.1-30.3) and remove force components vertically acting on the side elements via the rolls (30.1-30.3).

4. Heart-lung machine (2) according to any of claims 1 to 3, **characterised in that** a substantially enclosed space (36) is defined by the side elements (26.1, 26.2) and the connecting element (28).

5. Heart-lung machine (2) according to any of claims 1 to 4, **characterised in that** between two of the side elements (26.1, 26.2) a substantially planar and vertically extending cover (32.1, 32.2) is mounted which is preferably made of metal sheet.

6. Heart-lung machine (2) according to claim 4 and 5, **characterised in that** the cover (32.1, 32.2) encloses the space (36) in the direction of a horizontal plane and preferably comprises a flap (34) that can be opened.

7. Heart-lung machine (2) according to any of claims 4 to 6, **characterised in that** it comprises a connecting element (38) in the space (36) for connecting the medical device (24) or the medical devices, wherein the connecting element (38) is supported by a side element (26.1, 26.2) and preferably comprises a plurality of slots (40) for electronic plug-in cards.

8. Heart-lung machine (2) according to any of claims 1 to 7, **characterised in that** one or more of the side elements (26.1, 26.2) have a bar-shaped configuration or are bar-shaped.

## Revendications

1. Machine cardio-pulmonaire (2), comprenant
- une pluralité d'appareils médicaux (4.1-4.4, 24),
- un panneau de commande (6),
- deux ou plusieurs éléments latéraux s'étendant sensiblement verticalement (26.1, 26.2) et
- un élément de liaison (28) s'étendant entre les éléments latéraux (26.1, 26.2) et de préférence sensiblement horizontalement;
- dans lequel le panneau de commande (6) présente des éléments de commande séparés (10.1-10.4) pour la commande des appareils médicaux (4.1-4.4, 24) ;
- dans lequel l'élément de liaison (28) relie les uns aux autres les éléments latéraux (26.1, 26.2),
- dans lequel l'élément de liaison (28) et les éléments latéraux (26.1, 26.2) forment un cadre pour porter les appareils médicaux (4.1-4.4, 24), de sorte que les appareils médicaux (4.1-4.4, 24) puissent être reçus au moins en partie entre les éléments latéraux (26.1, 26.2), et
- dans lequel le panneau de commande (6) est conçu de façon à dépasser au-dessus d'une surface de base (12) des appareils médicaux (4.1-4.4, 24),
**caractérisée en ce que**
une position de commande remarquable (8) est **caractérisée en ce que** le panneau de commande (6) est conçu de telle manière que soit associé à chaque appareil (4.1-4.4, 24) un élément de commande (10.1- 10.4) aligné uniquement avec ledit appareil (4.1-4.4, 24) et avec la position de commande (8).

2. Machine cardio-pulmonaire (2) selon la revendication 1, **caractérisée en ce qu'**au moins l'un des éléments latéraux (26.1, 26.2) est étendu à plat.

3. Machine cardio-pulmonaire (2) selon la revendication 1 ou 2, **caractérisée en ce que** les éléments latéraux (26.1, 26.2) sont placés sur des rouleaux (30.1-30.3) et enlèvent des composantes de force agissant verticalement sur eux par le biais des rouleaux (30.1-30.3).

4. Machine cardio-pulmonaire (2) selon l'une des revendications 1 à 3, **caractérisée en ce que,** par les éléments latéraux (26.1, 26.2) et l'élément de liaison (28), un espace sensiblement fermé (36) est défini.

5. Machine cardio-pulmonaire (2) selon l'une des revendications 1 à 4, **caractérisée en ce que**, entre deux des éléments latéraux (26.1, 26.2), un recouvrement (32.1, 32.2) essentiellement plat et verticalement étendu, fait de préférence en tôle est aménagé.

6. Machine cardio-pulmonaire (2) selon la revendication 4 et 5, **caractérisée en ce que** le recouvrement (32.1, 32.2) entoure l'espace (36) en direction d'un plan horizontal et présente de préférence un volet (34) pouvant être ouvert.

7. Machine cardio-pulmonaire (2) selon l'une des revendications 4 à 6, **caractérisée en ce qu'**elle présente dans l'espace (36) un élément de raccordement (38) pour raccorder l'appareil médical (24) ou les appareils médicaux, dans laquelle l'élément de raccordement (38) est maintenu par un élément latéral (26.1, 26.2) et présente de préférence une multiplicité d'emplacements d'enfichage (40) pour des connecteurs enfichables électroniques.

8. Machine cardio-pulmonaire (2) selon l'une des revendications 1 à 7, **caractérisée en ce qu'**un ou plusieurs des éléments latéraux (26.1, 26.2) est ou sont formé(s) en forme de barre.
